# EUROPÄISCHE PATENTSCHRIFT

(11) **EP 1 526 826 B1**
(45) Veröffentlichungstag und Bekanntmachung des Hinweises auf die Patenterteilung: **17.10.2007**
(21) Anmeldenummer: 03784168.1
(22) Anmeldetag: 04.08.2003
(51) Int. Cl.: A61K 6/10

(54) **ZUBEREITUNGEN AUF AZIRIDINOPOLYETHERBASIS UND DEREN VERWENDUNG**
PREPARATIONS BASED ON AZIRIDINO POLYETHERS AND THE USE THEREOF
PREPARATIONS A BASE D'AZIRIDINOPOLYETHERS ET LEUR UTILISATION

(30) Priorität: 06.08.2002 DE 10235990
(43) Veröffentlichungstag der Anmeldung: 04.05.2005
(73) Patentinhaber: 3M ESPE AG, 82229 Seefeld (DE)
(72) Erfinder: KLETTKE, Thomas, 86911 Diessen (DE); FÜHRER, Cornelia, 87497 Wertach (DE); ECKERT, Adrian, 82211 Herrsching (DE); ECKHARDT, Gunther, 06231 Bad Dürrenberg (DE); WANEK, Erich, 86916 Kaufering (DE)
(86) Internationale Anmeldenummer: PCT/EP2003/008615
(87) Internationale Veröffentlichungsnummer: WO 2004/014323

(56) Entgegenhaltungen:
- DE-A- 1 544 837
- DE-A- 1 745 810
- DE-A- 10 026 852
- DE-A- 19 753 456
- US-A- 3 634 400

## Beschreibung

Die Erfindung betrifft monofunktionelle Aziridine enthaltende Zubereitungen auf der Basis von Aziridinopolymeren und ihre Verwendung zur Herstellung von Dentalmassen, insbesondere von Abformmassen.

Die Abformung der konkreten Verhältnisse im Mund des Patienten mit Hilfe geeigneter Abformmassen ist die Voraussetzung zur Herstellung von passgenauen Prothesen, Kronen und Brücken, Inlays und Onlays. Von den bekannten Abformmassen zeichnen sich die auf Aziridinopolyethern basierenden Massen durch ihren inhärent hydrophilen Charakter und ihre Fließeigenschaften im ausgehärteten Zustand aus, woraus sich eine hohe Präzision der Abdrücke ergibt.

Bei der Abformung der konkreten Verhältnisse im Mund des Patienten mit Hilfe geeigneter Abformmassen wirkt sich jedoch oft problematisch aus, dass der Vorgang des Abformens für den Patienten unangenehm ist. Aus diesem Grunde sollte die Verweildauer eines geeigneten Abformmaterials im Patientenmund nur einen kurzen Zeitraum in Anspruch nehmen. Eine zu frühe Entnahme des Abformmaterials kann jedoch zu einem Verlust an Genauigkeit und Zeichnungsschärfe im Hinblick auf die Abbildung der Mundverhältnisse führen, wenn die Abformmasse noch nicht ausreichend ausgehärtet ist.

Aus diesem Grunde wurden verschiedentlich Versuche unternommen, die Härtungszeit der eingesetzten Abformmassen, beispielsweise durch den Einsatz einer erhöhten Katalysatormenge oder durch unterschiedliche polymere Bindemittel, zu beschleunigen. Dies hatte jedoch in der Regel zur Folge, dass sich die Elastomereigenschaften der ausgehärteten Abformmasse verschlechterten.

Darüber hinaus müssen bei den aus dem Stand der Technik bekannten Abfonnmaterialien oft große Mengen an Weichmachern eingesetzt werden. Da die Weichmacher jedoch nicht chemisch in das polymere Netzwerk des ausgehärteten Abdrucks eingebunden sind, kann es bei der Lagerung der Abdrücke zu einer Migration der Weichmacher an die Oberfläche des Abdrucks kommen. Dadurch wird ein späteres Ausgießen der Abdrücke jedoch häufig nachteilig beeinflusst.

Es besteht daher ein Bedürfnis nach Abformmassen, die dem Zahnarzt bis zur Aushärtung der Abformmasse eine ausreichende Arbeitszeit belassen, jedoch im Patientenmund möglichst schnell zumindest soweit aushärten, dass die Verweildauer im Mund auf ein notwendiges Minimum verkürzt werden und die Behandlung damit für den Patienten angenehmer gestaltet werden kann. Weiterhin bestand ein Bedürfnis nach Abformmassen, die trotz eines geringeren Bedarfs an Weichmachern ausgezeichnete mechanische Eigenschaften und gute Langzeitstabilität aufweisen.

Die Herstellung von Aziridinogruppen tragenden Polymeren und ihre Verwendung in Dentalmaterialien ist seit langem bekannt. So beschreibt beispielsweise die DE 174 58 10 C die Herstellung von Formkörpern auf der Basis von Aziridinopolyethem. Dort wird zwar die Möglichkeit genannt, auch Abformmassen mit einem Gehalt an monofunktionellen Aziridinen einzusetzen, dies wird jedoch als nachteilig im Hinblick auf die mechanischen Eigenschaften bezeichnet. Ein Beschleunigungseffekt im Hinblick auf die Aushärtung der Dentalmassen wird in der angegebenen Druckschrift nicht beschrieben.

Die DE 100 26 852 A1 betrifft N-Alkyl-Aziridinoblockcopolymere, die ein Polysiloxangerüst tragen. Die Möglichkeit, eine Beschleunigung der Aushärtungszeit durch Zugabe monofunktioneller Aziridine zu erzielen, wird in der Druckschrift nicht offenbart.

Die Aufgabe der vorliegenden Erfindung bestand daher darin, Dentalmassen auf der Basis von Aziridinopolymeren bereitzustellen, die sich durch eine beschleunigte Aushärtung bei ausreichender Verarbeitungszeit (offener Zeit) auszeichnen. Eine weitere Aufgabe der Erfindung bestand insbesondere darin, Dentalmassen auf der Basis von Aziridinopolmeren bereitzustellen, die im Gegensatz zu aus dem Stand der Technik bekannten Dentalmassen schneller hohe Werte für die Shore-A Härte erreichen, wobei jedoch die vollständig ausgehärteten Massen in Bezug auf die aus dem Stand der Technik bekannten Dentalmassen in einem gewünschten Bereich liegende und im wesentlichen unveränderte Shore-A Härten aufweisen.

Diese Aufgabe wird gelöst durch Zubereitungen und daraus hergestellten Dentalmassen, wie sie im Rahmen des nachfolgenden Textes beschrieben sind.

Gegenstand der vorliegenden Erfindung ist daher eine Zusammensetzung, mindestens enthaltend zwei Komponenten Z1 und Z2, wobei die Zusammensetzung
a) als Komponente Z1 mindestens ein Polyadditionsprodukt oder ein Polykondensationsprodukt mit durchschnittlich 2 Aziridinogruppen oder mehr und einem Molekulargewicht von mindestens 1000 und
b) als Komponente Z2 mindestens eine Verbindung mit 1 Aziridinogruppe enthält, wobei mindestens eine Verbindung gemäß Komponente Z2 sich in ihrem chemischen Aufbau von mindestens einer Verbindung gemäß Komponente Z1 in mindestens einem weiteren Merkmal als der Zahl der Aziridinogruppen gemäß Anspruch 1 unterscheidet.

Unter einer "Zusammensetzung" wird im Rahmen des vorliegenden Textes ein Gemisch aus zwei oder mehr Verbindungen verstanden, wie es im Rahmen des vorliegenden Textes erläutert wird.

Eine erfindungsgemäße Zusammensetzung enthält mindestens zwei Komponenten Z1 und Z2, wobei Komponente Z1 ein Polyadditionsprodukt oder ein Polykondensationsprodukt mit durchschnittlich mindestens 2 Aziridinogruppen oder mehr und einem Molekulargewicht von vorzugsweise mindestens 1000 darstellt.

Der Begriff "Molekulargewicht" bezieht sich dabei auf das Zahlenmittel des Molekulargewichts wie es für die einzelnen Polymerklassen üblicherweise durch Gelpermeationschromatographie (GPC) gegenüber einem Standard mit definiertem Molekulargewicht ermittelt wird. Geeignete Messverfahren sind dem Fachmann bekannt.

Weiterhin lässt sich beispielsweise die Ermittlung der Molekulargewichte und der Verteilung der Molekulargewichte von polymeren Polyolen mittels Endgruppenbestimmung, beispielsweise durch kernresonanzmagnetische Methoden (NMR) durchführen. Ebenfalls zur Ermittlung der Molekulargewichte und der Verteilung der Molekulargewichte von polymeren Polyolen geeignet ist die Bestimmung der Hydroxylzahl wie sie beispielsweise in Houben-Weyl, "Methoden der organischen Chemie", 14/2, S. 17, Georg Thieme Verlag, Stuttgart, 1963 beschrieben wird. Weiterhin geeignet ist die in ASTM D2849 - Methode C beschriebene Verfahrensweise.

Ein besonders geeignetes Verfahren zur Bestimmung des Molekulargewichts (M_{w} und Mₙ) und der Molekulargewichtsverteilung organischer Diole lässt sich beispielsweise mittels GPC unter Verwendung einer Säulenkombination PSS SDV 10.000 Å + PSS SDV 500 Å + PSS SDV 100 Å mit einer Säulenabmessung von 8x300 mm und einer Korngröße von 5 µm durchgeführt. Als Vorsäure wird eine PSS SDV 100 Å mit einer Säulenabmessung von 8x50 mm und einer Korngröße von 10 µm eingesetzt. Als Laufmittel eignet sich besonders mit 200 ppm Jonol stabilisiertes THF bei einer Flussrate von 1,0 ml/min. Als Detektor wird ein Brechungsindexdetektor (RI) eingesetzt, das Injektionsvolumen der Proben (1 %ig m/m im Laufmittel eingewogen) beträgt 100 µl. Als Standardlösung wird eine Polystyrolstandardreihe (0,1 % m/m im Laufmittel eingewogen) eingesetzt. Die Auswertung erfolgt nach dem Prinzip der relativen GPC über ein automatisches Auswertemodul (TurboSEC Software) durch Vergleich der Elutionsvolumina der Probe mit den Elutionsvolumina der Polystyrolstandardreihe. Ausgewertet werden Mₙ, M_{w} und Polydispersität.

Als Polykondensationsprodukte eignen sich im Rahmen der vorliegenden Erfindung grundsätzlich alle durch Polykondensationsverfahren herstellbaren Polymeren, sofern sie die Anforderungen der Zusammensetzung im Hinblick auf deren bevorzugten Einsatz als Dentalmaterialen erfüllen. Geeignete Polykondensationsprodukte sind beispielsweise Polyester, Polyacetale oder Polysiloxane.

Von den bekannten Polyestern der verschiedenartigen Konstitutionen eignen sich zur Herstellung der Ausgangsprodukte besonders solche, die durch Polykondensation von Dicarbonsäuren mit Diolen oder durch Polykondensation von Oxycarbonsäuren erhältlich sind und im wesentlichen linear aufgebaut sind. Die Mitverwendung geringer Mengen tri- oder tetrafunktioneller Alkohole oder Carbonsäuren bei der Polykondensation ist möglich und in manchen Fällen für die mechanischen Eigenschaften der aus den Polyestern erhältlichen Zusammensetzungen im Hinblick auf deren Einsatz als Dentalmassen sogar vorteilhaft.

Als Polyole zur Herstellung der oben genannten Polyester kann eine Vielzahl von Polyolen eingesetzt werden. Beispielsweise sind dies aliphatische Alkohole mit 2 bis 4 OH-Gruppen pro Molekül. Die OH-Gruppen können sowohl primär als auch sekundär sein. Zu den geeigneten aliphatischen Alkoholen zählen beispielsweise Ethylenglykol, Propylenglykol, Butandiol-1,4, Pentandiol-1,5, Hexandiol-1,6, Heptandiol-1,7, Octandiol-1,8 und deren höhere Homologen oder Isomeren, wie sie sich für den Fachmann aus einer schrittweisen Verlängerung der Kohlenwasserstoffkette um jeweils eine CH₂-Gruppe oder unter Einführung von Verzweigungen in die Kohlenstoffkette ergeben. Ebenfalls geeignet sind höherfunktionelle Alkohole wie beispielsweise Glyzerin, Trimethylolpropan, Peritaerythrit sowie oligomere Ether der genannten Substanzen mit sich selbst oder im Gemisch aus zwei oder mehr der genannten Ether untereinander.

Weiterhin können als Polyole die Umsetzungsprodukte niedermolekularer polyfunktioneller Alkohole mit Alkylenoxiden, sogenannte Polyether, eingesetzt werden. Die Alkylenoxide weisen vorzugsweise 2 bis 4 C-Atome auf. Geeignet sind beispielsweise die Umsetzungsprodukte von Ethylenglykol, Propylenglykol, den isomeren Butandiolen oder Hexandiolen mit Ethylenoxid, Propylenoxid oder Butylenoxid, oder Gemischen aus zwei oder mehr davon. Ferner sind auch die Umsetzungsprodukte polyfunktioneller Alkohole, wie Glyzerin, Trimethylolethan oder Trimethylolpropan, Pentaerythrit oder Zuckeralkohole, oder Gemischen aus zwei oder mehr davon, mit den genannten Alkylenoxiden zu Polyetherpolyolen geeignet.

Entsprechende Polyether werden in dem Fachmann bekannter Weise durch Umsetzung der Startverbindung mit einem reaktiven Wasserstoffatom mit Alkylenoxiden, beispielsweise Ethylenoxid, Propylenoxid, Butylenoxid, Styroloxid, Tetrahydrofuran oder Epichlorhydrin oder Gemischen aus zwei oder mehr davon, umgesetzt.

Geeignete Startverbindungen sind beispielsweise Wasser, Ethylenglykol, Propylenglykol-1,2 oder -1,3, Butylenglykol-1,4 oder -1,3 Hexandiol-1,6, Octandiol-1,8, Neopentylglykol, 1,4-Hydroxymethylcyclohexan, 2-Methyl-1,3-propandiol, Glyzerin, Trimethylolpropan, Hexantriol-1,2,6, Butantriol-1,2,4 Trimethylolethan, Pentaerythrit, Mannitol, Sorbitol, oder Gemische aus zwei oder mehr davon.

Zur Herstellung entsprechender Polyester sind beispielsweise Bernsteinsäure, Adipinsäure, Korksäure, Azelainsäure, Sebacinsäure, Phthalsäure, Isophthalsäure, Terephthalsäure, Trimellithsäure, Phthalsäureanhydrid, Tetrahydrophthalsäureanhydrid, Hexahydrophthalsäureanhydrid, Tetrachlorphthalsäureanhydrid, Endomethylentetrahydrophthalsäureanhydrid, Glutarsäureanhydrid, Maleinsäure, Maleinsäureanhydrid, Fumarsäure, Dimerfettsäure oder Trimerfettsäure oder Gemische aus zwei oder mehr davon geeignet. Gegebenenfalls können untergeordnete Mengen an monofunktionellen Fettsäuren im Reaktionsgemisch vorhanden sein. Ebenfalls geeignet sind ungesättigte Dicarbonsäuren wie Maleinsäure oder Fumarsäure sowie aromatische Dicarbonsäuren, beispielsweise die isomeren Phthalsäuren, wie Phthalsäure, Isophthalsäure oder Terephthalsäure. Als Tricarbonsäuren sind beispielsweise Zitronensäure oder Trimellithsäure geeignet. Die genannten Säuren können einzeln oder als Gemische aus zwei oder mehr davon eingesetzt werden.

Die Polyester können gegebenenfalls einen geringen Anteil an Carboxylendgruppen aufweisen. Aus Lactonen, beispielsweise ε-Caprolacton oder Hydroxycarbonsäuren, beispielsweise ω-Hydroxycapronsäure, erhältliche Polyester, können ebenfalls eingesetzt werden.

Ebenfalls als Polyolkondensate geeignet sind Polyacetale. Unter Polyacetalen werden Verbindungen verstanden, wie sie aus Glykolen, beispielsweise Diethylenglykol oder Hexandiol oder deren Gemisch mit Formaldehyd erhältlich sind. Im Rahmen der Erfindung einsetzbare Polyacetale können ebenfalls durch die Polymerisation cyclischer Acetale erhalten werden.

Als Polysiloxane eignen sich grundsätzlich alle Polysiloxane, welche die geforderten Materialeigenschaften im Hinblick auf die bevorzugte Anwendung als Dentalmassen erfüllen. Besonders geeignet sind im Rahmen der vorliegenden Erfindung jedoch beispielsweise die Polysiloxangrundgerüste der in der DE 100 26 852 A1 Von S. 2, z. 55 bis S. 8, Z. 20 beschriebenen Aziridinogruppen tragenden Polysiloxane. Die Offenbarung der genannten Druckschrift wird als Bestandteil der Offenbarung des vorliegenden Textes betrachtet.

Als Polyadditionsprodukte eignen sich im Rahmen der vorliegenden Erfindung grundsätzlich alle durch Polyadditionsverfahren herstellbaren Polymeren, sofern sie die Anforderungen der Zusammensetzung im Hinblick auf deren bevorzugten Einsatz als Dentalmaterialen erfüllen. Geeignete Polyadditionsprodukte sind beispielsweise Polyurethane oder Polyether.

Als Polyurethane eignen sich im wesentlichen alle durch die Reaktion von Polyolen oder Polycarbonsäuren und Isocyanaten herstellbaren Polymeren. Entsprechende Herstellungsmethoden sind dem Fachmann bekannt, geeignete Polyole wurden im Rahmen des vorliegenden Textes bereits als Ausgangsstoffe zur Herstellung der o.g. Polyester beschrieben.

Im Rahmen einer bevorzugten Ausführungsform der vorliegenden Erfindung werden als Bestandteile der erfindungsgemäßen Zusammensetzungen Polyadditionsprodukte eingesetzt, bei denen es sich vorzugsweise um Polyether handelt.

Als Polyether eignen sich grundsätzlich alle Polyetherverbindungen, welche die geforderten Materialeigenschaften im Hinblick auf die bevorzugte Anwendung als Dentalmassen erfüllen. Geeignete Polyether und Verfahren zu deren Herstellung werden beispielsweise oben im Rahmen des vorliegenden Textes beschrieben. Besonders geeignet sind Polyetherverbindungen wie sie durch Polyaddition von Ethylenoxid, 1,2-Propylenoxid, 1,2-Butylenoxid oder Tetrahydrofuran oder von Gemischen aus zwei oder mehr der genannten Verbindungen unter Zuhilfenahme einer geeigneten Starterverbindung und eines geeigneten Katalysators erhältlich sind.

Besonders geeignet sind im Rahmen der vorliegenden Erfindung jedoch Polyetherverbindungen, die als Bestandteil mindestens eine von 1,2-Propylenglykol abgeleitete Wiederholungseinheit in der Polyetherkette aufweisen. So eignen sich als Polyethergrundgerüste für die in einer erfindungsgemäßen Zusammensetzung enthaltenen Aziridinogruppen tragenden Polymeren beispielsweise Polypropylenglykol oder Ethylenglykol/Propylenglykol-Copolymere oder Tetrahydrofuran/Propylenglykol-Copolymere oder Tetrahydrofuran/Ethylenglykol-Copolymere oder Gemische aus zwei oder mehr davon, insbesondere Polypropylenglykol oder Ethylenglykol/Propylenglykol-Copolymere oder Tetrahydrofuran/Ethylenglykol-Copolymere.

Geeignet sind beispielsweise Polyetherpolyole, die durch Copolymerisation von Tetrahydrofuran und Ethylenoxid im Molverhältnis 10 : 1 bis 1 : 1, vorzugsweise bis 3 : 1 in Gegenwart starker Säuren, beispielsweise Borfluorid-Etheraten, hergestellt werden.

Die zur Herstellung der Komponente Z1 einsetzbaren Polyetherpolyole weisen durchschnittlich mindestens 2 Hydroxylgruppen auf, können aber auch bis zu 20 Hydroxylgruppen pro Molekül, beispielsweise durchschnittlich bis zu etwa 3, 4, 5, 8, 10 oder 15 Hydroxylgruppen enthalten.

Die Molmassen (Mₙ) der Polyetherpolyole liegen üblicherweise im Bereich von 600 bis 20000 g/Mol, bevorzugt im Bereich von etwa 1000 bis 10000 g/Mol.

Die Verteilung der auf unterschiedlichen Monomeren basierenden Struktureinheiten im Polymeren kann dabei statistisch oder blockweise organisiert sein.

Geeignete Polyether werden darüber hinaus in der DE PS 1 745 810 beschrieben, deren diesbezügliche Offenbarung als Bestandteil der Offenbarung des vorliegenden Texts betrachtet wird.

Die im Rahmen der vorliegenden Erfindung als Komponente Z1 in den erfindungsgemäßen Zusammensetzungen enthaltenen Polymeren tragen durchschnittlich mindestens 2 Aziridinogruppen.

Der Begriff "durchschnittlich" ist dabei im Rahmen des vorliegenden Textes so auszulegen, dass ein Gemisch aus einer Vielzahl von Verbindungen der Komponente Z1 sowohl Verbindungen mit weniger als 2 Aziridinogruppen als auch Verbindungen mit mehr als 2 Aziridinogruppen aufweisen kann, wobei jedoch über die Gesamtzahl der Verbindungen der Komponente Z1 betrachtet die durchschnittliche Funktionalität aller Moleküle im Hinblick auf Aziridinogruppen 2 oder mehr beträgt.

Sämtliche genannten Typen von Polyadditions- oder Polykondensationsprodukten können durch beliebige, dem Fachmann bekannte Folgereaktionen mit Aziridinogruppen ausgestattet werden. So können beispielsweise zunächst Substituenten in ein entsprechendes Polymeres eingeführt werden, die ihrerseits zur Reaktion mit geeigneten Aziridinderivaten fähig sind. Häufig ist es möglich, cyclische Äther, vorzugsweise Epoxide, an die Kette so anzupolymerisieren, dass Produkte erhalten werden, die am Ende Substituenten enthalten, die mit Aziridin reagieren können. In Frage kommen beispielsweise Polyether, an die halogensubstituierte Epoxide, z. B. Epibromhydrin, anpolymerisiert sind. Diese Substanzen enthalten kurze halogensubstituierte Endgruppen, z. B. bei der Verwendung von Epibromhydrin-CH₂Br-Gruppen.

Bei Polyethern können deren OH-Endgruppen auch durch Halogenatome ersetzt werden, worauf die Halogenatome z. B. mit überschüssigem Ammoniak oder Phthalimidkalium in Aminogruppen übergeführt werden, die dann mit Ethyleniminocarbonestem zur Reaktion gebracht werden. Da die halogenhaltigen Polyether als Alkylierungsmittel wirken und deshalb unbeabsichtigte Vernetzungsreaktionen auslösen können, ist darauf zu achten, dass die Halogenatome möglichst weitgehend, z. B. durch Aminogruppen, ersetzt werden oder noch vorhandenes Resthalogen, beispielsweise durch Behandeln mit Alkalialkoholat, entfernt wird.

Ein weiteres Verfahren zur Herstellung der Aziridinogruppen tragenden Polymeren besteht in der Acylierung von OH-Gruppen tragenden Polyadditions- oder Polykondensationsprodukten mit Halogencarbonsäuren und anschließendem Umsatz mit entsprechenden Aziridinderivaten. Besonders geeignet sind hier α-Halogencarbonsäuren, z. B. Chloressigsäure, oder Brombuttersäure. Die Acylierung im Rahmen der beiden genannten Verfahren kann selbstverständlich auf verschiedene Weise erfolgen, z. B. durch säurekatalysierte Veresterung, durch Verwendung der Säureanhydride oder Säurechloride.

Ein weiterer Weg zu den erfindungsgemäß einsetzbaren Verbindungen ist der Umsatz von OH-Gruppen tragenden Polymeren mit wenigstens bifunktionellen Isocyanaten, vorzugsweise Diisocyanaten, z. B. 2,4-Toluylendiisocyanat, 4,4'-Diphenylmethandiisocyanat oder Naphthalin-1,5-diisocyanat. Bei dieser Umsetzung ist es zur Vermeidung von Nebenreaktionen häufig zweckmäßig, bei niederer Temperatur zu arbeiten, was durch hochaktive Katalysatoren wie tertiäre Amine, oder Metallverbindungen, wie Zinkacetylacetonat oder Organozinnverbindungen erreicht werden kann. Diese Katalysatoren stören im allgemeinen die anschließende Weiterverarbeitung der Substanzen nicht. Auch sogenannte isocyanatverlängerte Polyester, bei denen sich in der Kette Urethangruppen befinden, sind brauchbar.

Bei der Folgereaktion lässt man die mit endständigen Isocyanatgruppen versehenen Polyester mit geeigneten Alkyleniminderivaten reagieren. Hierfür bieten sich z. B. Ethyleniminderivate mit OH-Gruppen oder primären oder sekundären Aminogruppen an. Genannt seien Ethyleniminoamine, z. B. γ-Ethyleniminopropylamin und β-Ethyleniminoethylamin, ferner Ethyleniminoalkohole, wie 3-Ethyleniminopropanol-1, sowie die Ethyleniminoacylderivate von mindestens zweiwertigen Aminen. Die zuletzt genannte Substanzklasse besitzt unter anderem die Besonderheit, dass die daraus hergestellten Vor- und Endprodukte nur verhältnismäßig schwach basische Aminogruppen aufweisen.

Geeignete Möglichkeiten zur Ausstattung der Polymeren mit Aziridinogruppen werden beispielhaft in der DE PS 1 745 810 oder der DE 100 26 852 A1 genannt, wobei auf die genannten Druckschriften ausdrücklich Bezug genommen wird, und deren Offenbarung im Hinblick auf die Funktionalisierung von Polymeren mit Aziridinogruppen als Bestandteil der Offenbarung des vorliegenden Textes verstanden wird.

Als Komponente Z1 geeignete Polymere können die Aziridinogruppen endständig oder seitenständig oder endständig und seitenständig, vorzugsweise jedoch endständig tragen.

Die als Komponente Z1 einsetzbaren Aziridinopolymere sollten vorzugsweise eine dynamische Viskosität η von 10 bis etwa 500 Pa*s, insbesondere etwa 15 bis etwa 300 Pa*s (23 °C, gemessen mit einem Rotationsviskosimeter vom Typ CVO 120 HR der Firma Bohlin Instruments GmbH Pforzheim bei 23 °C, Platte-Platte-Geometrie, Plattendurchmesser: 20 mm oder Platte-Kegel-Geometrie, Scherrate 20 s⁻¹) aufweisen.

Ein bevorzugter Bereich der Viskosität liegt bei etwa 20 bis etwa 180 Pa*s bei 23° C.

Das Aziridinoäquivalent beträgt für die im Rahmen der vorliegenden Erfindung als Komponente Z1 eingesetzten Verbindungen etwa 250 bis etwa 25000 g/Äquivalent, insbesondere etwa 400 bis etwa 10.000 g/Äquivalent.

Eine erfindungsgemäß einsetzbare Komponente Z1 kann im Rahmen der vorliegenden Erfindung nur einen Typen an Aziridinopolymeren enthalten. Es ist jedoch ebenso möglich, dass eine erfindungsgemäß einsetzbare Komponente Z1 zwei oder mehr verschiedene Typen an Aziridinopolymeren enthält, beispielsweise 3, 4 oder 5 verschiedene Typen.

Unter einem "Polymertypen" wird dabei im Rahmen der vorliegenden Erfindung ein Polymeres verstanden, wie es sich bei der Polyaddition oder Polykondensation ausgewählter Monomerer unter den gewählten Reaktionsbedingungen ergibt. Ein Polymertyp kann daher Polymermoleküle unterschiedlicher chemischer Konstitution und unterschiedlichen Molekulargewichts umfassen, je nach gewählten Reaktionsbedingungen. Zwei Reaktionen, die mit identischen Monomerzusammensetzungen unter identischen Reaktionsbedingungen durchgeführt werden, führen jedoch erfindungsgemäß immer zu identischen Polymertypen. Zwei Reaktionen, die mit identischen Monomeren, jedoch unter unterschiedlichen Reaktionsbedingungen durchgeführt werden, können zu identischen Polymertypen führen, müssen dies jedoch nicht. Entscheidend ist dabei die Nachweisbarkeit von im Hinblick auf die Materialeigenschaften relevanten Unterschieden im Hinblick auf chemische Konstitution, Molekulargewicht und weiteren bestimmbaren Parametern. Zwei Reaktionen, die mit unterschiedlichen Monomerzusammensetzungen durchgeführt werden, führen erfindungsgemäß immer zu unterschiedlichen Polymertypen.

Im Rahmen einer bevorzugten Ausführungsform enthält eine erfindungsgemäß einsetzbare Komponente Z1 einen oder zwei verschiedene Typen von Aziridinopolymeren, insbesondere nur einen Typen.

Die oben beschriebenen Aziridinopolymeren können in der Regel in der im Rahmen der Herstellung angefallenen Form eingesetzt werden, sie können jedoch auch gereinigt werden, um besonders helle und hochwertige Produkte zu gewinnen. Hierfür kommen die üblichen Verfahren in Betracht, beispielsweise Filtration, gegebenenfalls in Lösung, über Kieselgur, Aluminiumoxyd, Behandlung mit Ionenaustauschern, Waschen der Lösungen in organischen Lösungsmitteln mit Wasser, wässrigem Alkohol, Salzlösungen u. dgl., sowie gegebenenfalls wiederholte Umfällung, beispielsweise aus aromatischen Lösemitteln oder Alkoholen. Ferner kann durch Fraktionierung in der üblichen Weise ein Reinigungseffekt erzielt werden. Außerdem sind so Produkte mit einheitlicherem Molekulargewicht erhältlich.

Vorzugsweise enthält eine erfindungsgemäße Komponente Z1 mindestens ein Aziridinopolymeres, das als Grundgerüst einen Polyether aufweist, vorzugsweise einen Polyether auf Basis von Polytetrahydrofuran (Poly-THF) oder ein Propylenglykol/Ethylenglykol Copolymeres oder ein Ethylenglykol / Tetrahydrofuran Copolymeres, unabhängig von der Herstellungsart.

Weitere zum Einsatz in Komponente Z1 geeignete Polyaziridinoverbindungen werden beispielsweise in der Offenlegungsschrift der DE 15 44 837, S. 3 - S. 14 genannt.

Eine erfindungsgemäße Zusammensetzung enthält als Komponente Z2 mindestens eine monofunktionelle Aziridinoverbindung, die sich in ihrem chemischen Aufbau von mindestens einer Verbindung gemäß Komponente Z1 in mindestens einem weiteren Merkmal als der Zahl der Aziridinogruppen unterscheidet.

Als unterscheidende Merkmale eignen sich grundsätzlich alle Merkmale, die über die Zahl der Aziridinogruppen hinaus eine eindeutige Unterscheidung mindestens einer Verbindung gemäß Komponente Z1 von mindestens einer Verbindung gemäß Komponente Z2 erlauben. Geeignete unterscheidende Merkmale sind beispielsweise Molekulargewicht oder chemische Konstitution.

So können als monofunktionelle Aziridinoverbindungen beispielsweise Verbindungen eingesetzt werden, deren durchschnittliches Molekulargewicht niedriger oder höher ist als das durchschnittliche Molekulargewicht der in Komponente Z1 vorliegenden Polymeren. Ebenso können als monofunktionelle Aziridinoverbindungen beispielsweise Verbindungen eingesetzt werden, deren chemischer Aufbau sich von mindestens einer im Rahmen der Komponente Z1 eingesetzten polyfunktionellen Verbindung unterscheidet.

Wenn als monofunktionelle Aziridinoverbindungen Polymere eingesetzt werden, so sollte es sich bei diesen Polymeren vorzugsweise um Polymere eines anderen Polymertyps als mindestens eines der im Rahmen von Komponente Z1 eingesetzten Polymeren handeln.

Es ist beispielsweise vorgesehen, dass es sich bei der monofunktionellen Aziridinoverbindung um eine niedermolekulare Aziridinoverbindung mit einem durchschnittlichen Molekulargewicht von weniger als etwa 300 oder um eine höhermolekulare Aziridinoverbindung mit einem durchschnittlichen Molekulargewicht von etwa 300 oder mehr als etwa 300 handelt.

Als monofunktionelle Aziridinoverbindungen eignen sich beispielsweise Verbindungen, wie sie sich durch Umsetzung niedermolekularer Verbindungen, die eine funktionelle Gruppe aufeisen die mit mindestens einem der bereits oben im Rahmen der Beschreibung der Funktionalisierung der Polymeren genannten Aziridinoderivate reagieren kann, erhalten lassen. Geeignet sind beispielsweise Aziridinoderivate, wie sie sich durch Umsetzung niedermolekularer linearer oder verzweigter, gesättigter oder ungesättigter oder cyclischer oder aromatischer Alkohole, Epoxide oder Carbonsäuren mit Aziridinoderivaten, beispielsweise γ-Ethyleniminopropylamin oder 3-Ethyleniminopropanol-lerhalten lassen. Ebenfalls zur Funktionalisierung geeignet sind die mit entsprechenden funktionellen Gruppen versehenen im Hinblick auf Aziridinogruppen monofunktionelle Verbindungen, wie sie in der DE 100 26 852 A1 auf S. 4, Z. 20 bis S. 7, Z. 42 genannt werden.

Geeignete niedennolekulare Alkohole sind beispielsweise Methanol, Ethanol, die isomeren Propanole, Butanole, Pentanole Hexanole, Heptanole oder Octanole, Cycloalkanole mit 6 bis etwa 44 C-Atomen oder aromatische Alkohole mit 6 bis etwa 22 C-Atomen. Besonders geeignet sind die Aziridinoderivate des Butanols.

Geeignete Verbindungen zur Herstellung von monofunktionellen Aziridinoderivaten mit einem Molekulargewicht von mehr als etwa 300, wie sie sich als Bestandteil der erfindungsgemäßen Komponente Z2 einsetzen lassen, sind beispielsweise monofunktionelle Polymere wie sie oben bereits beschrieben wurden. Es ist dabei möglich, beispielsweise den gleichen Polymertyp einzusetzen wie er in Komponente Z1 bereits vorliegt. Es ist jedoch ebenfalls möglich einen Polymertypen einzusetzen, der sich von einem oder mehreren in Komponente Z1 vorliegenden Polymertypen unterscheidet.

Im Rahmen einer bevorzugten Ausführungsform der vorliegenden Erfindung weist eine als Bestandteil von Komponente Z2 eingesetzte Aziridinoverbindung keine OH-, SH, NH- oder NR-Gruppe, worin R für einen linearen oder verzweigten, gesättigten oder ungesättigten Alkylrest mit 1 bis etwa 44 C-Atomen steht, auf.

Besonders als monofunktionelle Aziridinoverbindungen mit einem Molekulargewicht von mehr als etwa 300 geeignet sind Polyadditions- oder Polykondensationsprodukte mit einer Aziridinogruppe, beispielsweise Polyesteraziridine oder Polyetheraziridine, vorzugsweise Polyetheraziridine.

Es kann sich bei den bevorzugten Polyetheraziridinen beispielsweise um Homopolyether handeln, es können jedoch auch Copolyether eingesetzt werden. Besonders bevorzugt ist im Rahmen der vorliegenden Erfindung der Einsatz von im Hinblick auf Aziridinofunktionen monofunktionellem Polypropylenglykol. Besonders bevorzugt ist dabei der Einsatz von Polyethern, deren Kettenende keine OH-, SH, NH- oder NR-Gruppe als terminale Gruppe aufweist. Geeignet sind beispielsweise Polyether, die terminal jeweils eine Aziridinogruppe und eine Alkylgruppe, eine Estergruppe, eine Amidgruppe oder dergleichen aufweisen. Besonders geeignet sind Polyetherverbindungen, die an einem Ende über eine Ethergruppe mit einer linearen oder verzweigten gesättigten Alkylgruppe mit 1 bis 8 C-Atomen, insbesondere 2 bis 6 C-Atomen, verschlossen sind.

Es hat sich gezeigt, dass der Anteil der Komponente Z2 an der erfindungsgemäßen Zusammensetzung in Bezug auf den Anteil an Komponente Z1 so gewählt werden sollte, dass das Verhältnis von Aziridinogruppen aus Komponente Z1 zu Aziridinogruppen aus Komponente Z2 etwa 20:1 bis etwa 1:1, beispielsweise etwa 10:1 bis etwa 1,2:1 oder etwa 5:1 bis etwa 1,5:1 gewählt werden sollte.

Vorzugsweise beträgt der auf das Gewicht der Komponenten Z1 und Z2 bezogene Anteil an Komponente Z2 an der erfindungsgemäßen Zusammensetzung mindestens etwa 0,1 Gew.-% und höchstens etwa 40 Gew.-%, beispielsweise etwa 0,2 bis etwa 35 oder etwa 0,3 bis etwa 30 oder etwa 0,4 bis etwa 25 oder etwa 0,5 bis etwa 20 Gew.-%. Die oben genannten Werte variieren üblicherweise mit dem Molekulargewicht der eingesetzten Komponente Z2.

Die Konsistenz einer erfindungsgemäßen Zusammensetzung liegt vorzugsweise innerhalb eines Bereichs von etwa 20 bis etwa 50 mm Scheibendurchmesser, insbesondere etwa 25 bis etwa 45 mm Scheibendurchmesser, gemessen entsprechend EN ISO 4823:2000.

Neben den Komponenten Z1 und Z2 kann eine erfindungsgemäße Zusammensetzung noch einen oder ein Gemisch aus zwei oder mehr Zusatzstoffen enthalten.

Geeignete Zusatzstoffe sind beispielsweise Verbindungen, die eine Weichstellung der ausgehärteten Dentalmassen-Zusammensetzungen bewirken. Solche Verbindungen können sowohl typische Weichmacher sein, wie sie auch für andere polymere Systeme angeboten werden, wie Ester von mehrwertigen Carbonsäuren, polyaromatische Verbindungen und Sulfonsäureester oder Verbindungen, die außer der Weichstellung auch andere Effekte wie beispielsweise Tensidwirkung, Erhöhung der Standfestigkeit und Verbesserung des Fließverhaltens bewirken.

Typische Weichmacher sind beispielsweise Verbindungen vom Estertyp wie C12- bis C15-Alkyllactate, Ethyl- oder Butylester der Citronensäure oder der Acetylcitronensäure, Phthalsäureester längerer verzweigter Alkohole, wie Bis(2-ethylhexyl)phthalat oder Phthalsäurepolyester, C₂ bis C₂₂-Dialkylester von C₂ bis C₆-Dicarbonsäuren wie Bis(2ethylhexyl)-adipat, Dioctylmaleat, Diisopropyladipat, aromatische und aliphatische Sulfonsäureester wie C₂ bis C20-Alkylsulfonsäureester des Phenols oder von C₁ bis C₂₂-Alkanolen oder typische aromatische Weichmacher wie Polyphenyle in einem weiten Viskositätsbereich, einschließlich wachsartiger Polyphenyle wie sie beispielsweise von der Fa. Monsanto erhältlich sind, Dibenzyltoluol, Isomerengemische von C₂₀ bis C₄₀-Aromaten, wobei die Verwendung von Gemischen aus Weichmachern des Estertyps und des aromatischen Typs bevorzugt ist.

Ein Beispiel für ein bevorzugtes Weichmachergemisch ist ein Gemisch aus Acetyltributylcitrat und Dibenzyltoluol.

Ebenfalls als Zusatzstoffe geeignet sind Trisacylester des Glyzerins nicht tierischen Ursprungs. Geeignete Zusatzstoffe können beispielsweise aus modifizierten Fetten pflanzlichen Ursprungs wie aus hydriertem Palmöl oder Sojaöl oder aus synthetischen Fetten bestehen.

Geeignete Fette sind in der DE 197 11 514 A1 (z. B.: S. 2, Z. 65 - S. 3, Z. 22) beschrieben, auf die hier vollinhaltlich Bezug genommen wird. Besonders geeignet sind Avocadoöl, Baumwollsaatöl, Erdnussöl, Kakaobutter, Kürbiskernöl, Leinöl, Maiskeimöl, Olivenöl, Palmöl, Reisöl, Rüböle, Saffloröl, Sesamöl, Sojaöl, Sonnenblumenöl, Traubenkernöl, Weizenkeimöl, Borneotalg, Fulwatalg, Hanföl, Illipebutter, Lupinienöle, Kandelnussöl, Kapoköl, Katiaufett, Kenafsamenöl, Kekunaöl, Mohnöl, Mowrahbutter, Okraöl, Perillaöl, Salbutter, Sheabutter und Tungöl, sofern diese Fette vor ihrer Verwendung gehärtet wurden. Als geeignet gehärtete Fette werden solche betrachtet, deren Jod-Zahl (gemessen nach der Norm DGF C-V 11 Z2 kleiner als 20 ist. Besonders bevorzugt sind Fette, deren Jod-Zahl kleiner als 5 ist. Die Durchführung von Fetthärtungen ist beispielsweise in "Ullmanns Enzyklopädie der industriellen Chemie", 4. Aufl., Band 11, S. 469 beschrieben.

Ebenso verwendbar sind Mischungen dieser natürlich vorkommenden Fette, sowie künstlich hergestellte Fette, wie Softisan 154 oder Dynasan 118 (Fa. Hüls). Die Herstellung derartiger künstlicher Triacylglyceride ist für den Fachmann relativ einfach und kann beispielsweise aus Glyzerin und den entsprechenden Fettsäuremethylestern erfolgen. Derartige Veresterungsreaktionen sind u. a. in "Houben-Weyl, Methoden der Organischen Chemie", Bd. E5/Teil 1, S. 659 ff. beschrieben.

Bevorzugte Triacylglyceride entsprechen der Formel:

**R²-O-CH₂-CH(OR¹)-CH₂-O-R³**

in welcher R¹, R² und R³ unabhängig voneinander C₁₁H₂₃CO, C₁₃H₂₇CO, C₁₅H₃₁CO oder C₁₇H₃₅CO bedeuten. Auch Gemische solcher Triacylglyceride kommen in Betracht.

Ebenfalls als Zusatzstoffe geeignet sind flüssige polymere Verbindungen mit Molmassen von mehr als etwa 2000 g/Mol, beispielsweise Verbindungstypen, wie Polyether, Polyester, Polyurethane, Polycarbonate oder Polyolefine, wobei als Endgruppen Hydroxyl-, Ether-, Alkyl- und Acylgruppen geeignet sind.

Eine spezielle Verbindungsklasse von flüssigen Polymeren stellen solche vom Polyethertyp dar.

Hierbei zeichnen sich solche Polyether besonders aus, welche die gleiche oder eine ähnliche Molmasse besitzen, wie die in Bestandteil Z1 verwendeten Aziridinopolyether.

Bis-Hydroxyl- oder Bis-Acetyl-polyether aus Oxytetramethylen- und Oxydimethylen-Einheiten im Verhältnis 4 : 1 bis 3 : 1 und Molmassen im Bereich von 3000 bis 8000 g/Mol sind beispielsweise als Zusatzstoffe geeignet.

Im Gemisch mit den genannten Polyethern oder auch als alleinige Zusatzstoffe können auch Polypropylenoxidpolyole und/oder Copolymerisate und/oder Blockcopolymerisate von Ethylenoxid und Propylenoxid mit Hydroxyl- oder Acetyl-Endgruppen eingesetzt werden.

Bei den Blockcopolymerisaten mit Molmassen größer 2000 g/Mol kann zusätzlich die lösungsvermittelnde Wirkung dieser tensidartigen Verbindungen genutzt werden.

Weiterhin kann durch die Wahl und die Mischung der vorgenannten Polyetherderivate das Fließverhalten und die notwendige Einstellung von Hydrophilie und Hydrophobie der gemischten Zubereitungen entscheidend beeinflusst werden.

Die erfindungsgemäßen Zusammensetzungen können weiterhin als Zusatzstoffe 10 bis 15 Gew.-% an verstärkend wirkenden Füllstoffen enthalten.

Für diesen Zweck können organische und anorganische Feststoffe eingesetzt werden, die in den erfindungsgemäßen Zusammensetzungen während der Lagerung keine unerwünschten Reaktionen hervorrufen und nach der Mischung der getrennt gelagerten Komponenten den Abbindeverlauf nicht beeinträchtigen. Zu diesem Zweck sind beispielsweise Kieselsäure, Zinkoxyd, Calciumcarbonat, Bariumsulfat, Quarzmehl, Schwerspat, Flussspat, Calciumphosphat oder Kaolin geeignet.

Besonders bewährt haben sich dabei Füllstoffe mit einem SiO₂-Antei von mehr als etwa 50 Gew.-%, beispielsweise mindestens etwa 90 Gew.-%, wie Quarzmehl und feinteilige Kieselsäuren synthetischen oder natürlichen Ursprungs.

Geeignet sind beispielsweise sind pyrogene Kieselsäuren und Fällungskieselsäuren, die meist in oberflächenmodifizierter Form eingesetzt werden sowie Diatomeenerde unterschiedlicher Fundstellen.

Gemische von aufbereiteter Diatomeenerde mit einem pH-Wert der 5 %-igen wässrigen Suspension von 8 bis 10 und pyrogener, oberflächenmodifizierter Kieselsäure mit BET-Oberflächen von 100 bis 600 m²/g sind besonders geeignet.

Weiterhin können die erfindungsgemäßen Zubereitungen weitere Zusatzstoffe wie Farbstoffe und Farbpigmente, Desinfektionsmittel, Aromen oder Geschmackskorrigentien enthalten.

Weiterhin können als Zusatzstoffe Säurefänger eingesetzt werden, die in den Ausgangsmaterialien enthaltene Säuren, saure Gruppen oder säureabspaltende Substanzen durch neutralisieren. Hierfür geeignet sind beispielsweise Amine, insbesondere tertiäre Amine.

Weiterhin können als Zusatzstoffe Modifikatoren eingesetzt werden, wie sie in der DE 32 45 052 (S. 4 - S. 6) beschrieben werden, wobei auf die dort genannten Modifikatoren ausdrücklich Bezug genommen wird und diese als Bestandteil der Offenbarung des vorliegenden Textes verstanden werden.

Die oben genannten Zusatzstoffe sind in einer erfindungsgemäßen Zusammensetzung üblicherweise in einer Menge von 0 bis etwa 60 Gew.-%, beispielsweise etwa 10 bis etwa 40 Gew.-% enthalten.

Die erfindungsgemäßen Zusammensetzungen lassen sich prinzipiell auf beliebige, dem Fachmann bekannte Weise erhalten. Beispielsweise werden die einzelnen Bestandteile der Zusammensetzung in einer oder mehreren aufeinanderfolgenden Stufen unter Verwendung geeigneter Mischgeräte miteinander vermischt. Gegenstand der vorliegenden Erfindung ist daher auch ein Verfahren zur Herstellung einer erfindungsgemäßen Zusammensetzung, bei dem zwei Komponenten Z1 und Z2 vermischt werden, wobei
a) als Komponente Z1 mindestens ein Polyadditionsprodukt oder ein Polykondensationsprodukt mit durchschnittlich 2 Aziridinogruppen oder mehr und einem Molekulargewicht von mindestens 1000 und
b) als Komponente Z2 mindestens eine Verbindung mit 1 Aziridinogruppe eingesetzt wird, und
wobei mindestens eine Verbindung gemäß Komponente Z2 sich in ihrem chemischen Aufbau von mindestens einer Verbindung gemäß Komponente Z1 in mindestens einem weiteren Merkmal als der Zahl der Aziridinogruppen gemäß Anspruch 7 unterscheidet.

Eine erfindungsgemäße Zusammensetzung eignet sich zur Herstellung von Dentalmassen.

Gegenstand der vorliegenden Erfindung ist daher auch eine Dentalmasse, umfassend mindestens eine Basiskomponente B und eine Katalysatorkomponente K, wobei Komponente B mindestens eine erfindungsgemäße Zusammensetzung und Komponente K mindestens einen Katalysator zur Vernetzung zumindest eines Teils der Komponente B enthält.

Zur Herstellung einer erfindungsgemäßen Dentalmasse wird eine erfindungsgemäße Zusammensetzung als Basiskomponente B mit einer Katalysatorkomponente K versetzt.

Die Katalysatorkomponente K enthält mindestens eine Startersubstanz, welche die Polymerisation der Aziridinogruppen tragenden Bestandteile der Komponente B auslöst und damit eine Aushärtung der gesamten Dentalmasse bewirkt.

Als Startersubstanz kommen grundsätzlich alle für Aziridine polyrnerisationsauslösenden Verbindungen in Betracht, sofern sie eine geeignete Abbindegeschwindigkeit und geeignete Elastomereigenschaften für die ausgehärtete Dentalmasse erzielen.

So sind für die Verwendung in zweikomponentigen Abformmassen, die auf den vorstehend beschriebenen Polyetherderivaten beruhen, solche Startersubstanzen geeignet, die eine Aushärtung bei Raumtemperatur der gemischten Zubereitung in einem Zeitraum von 1 bis 20 Minuten zu einem elastischen Festkörper ermöglichen, wobei dieser Festkörper die Anforderungen an eine elastische Abformmasse gemäß DIN/EN 2482 erfüllt und eine Shore A-Härte (DIN 53 505) von mindestens 20 nach 24 Stunden Lagerzeit besitzt.

Eine erfindungsgemäße Dentalmasse wird im Rahmen einer bevorzugten Ausführungsform der vorliegenden Erfindung im Hinblick auf die Komponenten B und K so eingestellt, dass sie nach Mischen der Basiskomponente B und der Katalysatorkomponente K bei Raumtemperatur innerhalb eines Zeitraums von 20 Minuten oder weniger eine Shore-A Härte von mindestens 80% des nach 24 Stunden erreichten Werts für die Shore-A Härte aufweist.

Als Startersubstanzen in einer erfindungsgemäß geeigneten Katalysatorkomponente K können grundsätzlich alle bekannten Starter eingesetzt werden. Zweckmäßigerweise verwendet man solche Starter bzw. Startersysteme, die eine einfache Einstellung des Aushärtungsverlaufs zulassen, keine Nebenwirkungen erzeugen und die reproduzierbare Erreichung der erforderlichen Niveaus der mechanischen Eigenschaften ermöglichen.

Eine zusammenfassende Darstellung der für die Aushärtung von N-Alkylaziridinoverbindungen verwendeten Startersubstanzen ist beispielsweise in O. C. DERMER, G. E. HAM, "Ethylenimine and other Aziridines" Academic Press (1969) enthalten.

Besonders als Startersubstanzen geeignet sind Trisalkylsulfbniumsalze wie sie beispielsweise in der US 4,167,618 (z. B.: Sp. 2, Z. 36 - Sp. 4, Z. 32 sowie Beispiele) beschrieben werden. Die genannten Trisalkylsulfoniumsalze werden als Bestandteil der Offenbarung des vorliegenden Textes verstanden.

In der Patentschrift DE 914 325 wird die Verwendung von Oxonium-, Ammonium- und Sulfoniumsalzen als Startersubstanzen vorgeschlagen (z.B.: S. 2, Z. 77 - S. 3, Z. 100 sowie Beispiele), wobei die dort genannten Startersubstanzen ebenfalls als Bestandteil der Offenbarung des vorliegenden Textes angesehen werden.

In der Patentanmeldung DE 100 18 918 A1 werden Starter beschrieben, die der Katalysatorkomponente einen lediglich geringen Säuregrad verleihen und die eine gut einstellbare, relativ lange Verarbeitungszeit nach erfolgter Mischung von Basiskomponente und Katalysatorkomponente ermöglichen. Auch auf die dort genannten Verbindungen wird ausdrücklich verwiesen und die dort genannten Startersubstanzen werden ebenfalls als Bestandteil der Offenbarung des vorliegenden Textes angesehen.

Besonders geeignet sind die in der US 4,176,618 genannten Starterverbindungen. Die auf Startersubstanzen bezogene Offenbarung dieser Druckschrift wird als Bestandteil der Offenbarung des vorliegenden Textes angesehen.

Startersysteme dieses Typs sind geeignet, die erfindungsgemäßen Basiskomponenten in der notwendigen Geschwindigkeit auszuhärten. Durch ihre Verwendung sind die gewünschten Eigenschaften des elastischen Festkörpers erreichbar.

Die Patentanmeldung DE-199 42 459 beschreibt Elastomermassen mit verbesserter Katalysatorkomponente, die sich durch eine erhöhte Dehnbarkeit auszeichnen. Gemäß dieser Erfindung werden Borsäurekomplexe als Starter eingesetzt. Diese Starter haben sich für die Aushärtung der N-Alkylaziridinopolymeren gemäß vorliegender Erfindung ebenfalls bewährt und können im Rahmen der vorliegenden Erfindung eingesetzt werden.

Vorzugsweise werden im Rahmen der vorliegenden Erfindung die nachfolgenden Starterverbindungen eingesetzt: das Zink-Salz der p-Toluolsulfonsäure, β-(S-Lauryl-S-ethylsulfonium)butyronitriltetrafluoroborat, Dodecylbenzolsulfonsäurezinksalz, β-(S-Lauryl-S-ethylsulfonium)-β-phenylacrylsäurebutylestertetrafluoroborat.

Eine erfindungsgemäß eingesetzte Katalysatorkomponente K kann neben einer der oben genannten Starterverbindungen oder einem Gemisch aus zwei oder mehr davon noch einen oder mehrere Zusatzstoffe enthalten. Als Zusatzstoffe eignen sich die bereits oben im Rahmen des vorliegenden Textes genannten Zusatzstoffe.

Die Starterverbindungen der Komponente K können beispielsweise leicht bewegliche Flüssigkeiten oder Feststoffe darstellen, deren gleichmäßige Einarbeitung in die mehr oder weniger viskosen Massen der Komponente B Schwierigkeiten schwierig sein kann. Zur Vermeidung dieses Nachteils können die Starterverbindungen in eine den jeweils beabsichtigten Anwendungsgebieten entsprechende viskose Form gebracht werden, z. B. durch Einarbeitung von Füllmitteln mit großer Oberfläche, wie hochdisperse Kieselsäure.

Auch die Verwendung von Lösungen der Starterverbindungen in geeigneten Weichmachern als Komponente K ist oft zweckmäßig; auf diese Weise werden nicht nur extreme Mischungsverhältnisse vermieden, sondern es können auch bei Raumtemperatur feste Vernetzungsmittel, z. B. Acetyltributylcitrat, bequem in die Komponente K eingearbeitet werden.

Beispielsweise enthält eine erfindungsgemäß geeignete Komponente K mindestens eine der nachfolgenden Klassen von Verbindungen, die eine Weichstellung der ausgehärteten Dentalmassen bewirken, nämlich
A typische Weichmacher mit Molmassen kleiner 500 g/Mol,
B bei Raumtemperatur feste Trisacylglyceride mit Molmassen im Bereich von 500 bis 2000 g/Mol oder
C bei Raumtemperatur flüssige Polymere mit Molmassen über 2000 g/Mol,
oder ein Gemisch aus zwei oder mehr davon.

Die Starterverbindungen sind in der Komponente K in der Regel in einer Menge von 0,5 bis 90 Gewichtsprozent, beispielsweise von 2 bis 80 Gewichtsprozent oder etwa 5 bis etwa 50 Gew.-% , enthalten.

Zur Herstellung der erfindungsgemäßen Dentalmassen werden die Komponenten B und K in geeigneten Mengenverhältnissen vermischt.

Vorteilhaft ist die praktische Durchführung der Mischung mittels kontinuierlicher Mischer, meist bestehend aus einem die Komponenten B und K enthaltenden Vorratsgefäß und statischen oder dynamischen Mischelementen.

Mit diesen Geräten ist eine ausreichende Mischgüte erreichbar, was leicht an der gleichmäßigen Färbung kontrolliert werden kann.

Üblicherweise wird bei Dentalmassen auf Polyetherbasis das Volumenmischverhältnis oder das Gewichtsverhältnis zwischen Katalysatorkomponente K und Basiskomponente B auf Werte von etwa 3 : 1 bis etwa 1 : 10 eingestellt, wobei die Einstellungen von etwa 1 : 2 bis etwa 1 : 5, insbesondere etwa 1 : 2 oder etwa 1 : 5, besonders bevorzugt sind.

Das Molverhältnis von Aziridinogruppen zu Anionen der Starterverbindungen beträgt im Rahmen der erfindungsgemäßen Dentalmassen etwa 3:1 bis etwa 0,9:1, beispielsweise etwa 2:1 bis etwa 1:1, insbesondere etwa 1,8:1 bis etwa 1,2:1.

Aus den genannten Werten ergibt sich entsprechend, dass die Zusammensetzung der Komponenten K und B in Abhängigkeit vom gewünschten Molverhältnis der reaktiven Aziridinogruppen zu den Anionen der Starterverbindung oder des Gemischs aus zwei oder mehr Starterverbindungen innerhalb weiter Bereiche variiert werden kann.

Die erfindungsgemäßen Dentalmassen auf Polyetherbasis mit verbesserter Entformbarkeit und verbessertem Anfließverhalten werden beispielsweise aus Zubereitungen erhalten, die insgesamt etwa
- 30 bis 80 Gew.-%, bevorzugt 45 bis 71 Gew.-% von Aziridinogruppen tragenden Verbindungen;
- 8 bis 40 Gew.-%, bevorzugt 10 bis 25 Gew.-% von Verbindungen, die eine Weichstellung der ausgehärteten Dentalmassen bewirken;
- 4 bis 25 Gew.-%, bevorzugt 9 bis 16 Gew.-% Füllstoffe;
- 4 bis 20 Gew.-%, bevorzugt 4 bis 16 Gew.-% an weiteren Wirkstoffen, wie Farbmitteln, Aromen, Startern, Verzögerern, Beschleunigern, rheologischen Additiven, Konsistenzgebern und Tensiden;

Besonders geeignete Komponenten und Fonnulierungen werden beispielsweise in den folgenden Patenten und Patentanmeldungen beschrieben: DE 1745810, US 3453242, US 1544837, US 4167618, DE 3245052, DE 3728216, EP 0421371, DE 4306997, DE 4321257, DE 19753461, DE 19740234, DE 10001747, DE 10018918.

Der Einsatz der erfindungsgemäßen Zubereitungen kann in sehr unterschiedlichen zahnmedizinisch oder zahntechnisch verwendeten Dentalmassen erfolgen. Bevorzugte Einsatzgebiete solcher Dentalmassen sind die einphasige und die zweiphasige zahnmedizinische Abformung und die Bissregistrierung.

Gegenstand der Erfindung sind auch Behältnisse und Mischvorrichtungen, enthaltend aus den erfindungemäßen Zubereitungen hergestellte Massen, insbesondere Dentalmassen, wie Kartuschen, Beutel, Abformlöffel, statische und dynamische Mischer bzw. Mischgeräte.

Gegenstand der Erfindung ist auch ein Kit zur Herstellung von Dentalmassen, umfassend mindestens eine erfindungsgemäße Zusammensetzung als Komponente Z1 und mindestens eine einen Katalysator zur Vernetzung zumindest eines Teils der Komponente B enthaltende Komponente K, wobei die Komponenten B und K voneinander getrennt vorliegen.

Ein weiterer Gegenstand der vorliegenden Erfindung ist die Verwendung einer erfindungsgemäßen Zusammensetzung als Basiskomponente B für Beschichtungen, Abformmassen, Dichtungen oder Dentalabdruckmassen.

Ein weiterer Gegenstand der vorliegenden Erfindung ist die Gegenstand der vorliegenden Erfindung ist auch die Verwendung einer Verbindung mit 1 Aziridinogruppe zur Beschleunigung der Abbindegeschwindigkeit von erfindungsgemäßen Dentalmassen.

Die Erfindung wird nachfolgend durch Ausführungsbeispiele näher erläutert.

### Ausführungsbeispiele:

### Messungen:

Die Messung der Shore-A Härte ist eine dem Fachmann bekannte Methode, um den Grad der Aushärtung zu messen. Zeitabhängige Messungen wurden gemäß DIN 53505 durchgeführt.

Die rheologischen Untersuchungen wurden mit einem Rotationsviskosimeter vom Typ CVO 120 HR der Firma Bohlin Instruments GmbH Pforzheim durchgeführt. Die Messungen erfolgten bei 23 °C. Verwendet wurde eine Platte-Platte-Geometrie, der Plattendurchmesser betrug 20 mm. Bestimmt wurde die dynamische Viskosität η in Abhängigkeit von der Scherrate γ̇.

Die unten aufgeführten Reißfestigkeiten und Dehnungswerte wurden in Anlehnung an die DIN 50125, Form B durchgeführt. Als Prüfgerät diente eine Universalprüfmaschine Zwick 1435 der Firma Zwick GmbH & Co Ulm. Die Prüfform zur Herstellung der Prüfkörper besteht aus 2 Halbformen aus Messing nach DIN 50125, Form B, hat einen Durchmesser von 6,0 ± 0,1 mm, und eine Messlänge von 50,0 ± 0,1 mm (Zugprobe B 6 x 50 DIN 50125).

Bei der Probenvorbereitung werden Basis- und Katalysatorkomponente im definierten Volumenverhältnis 5 : 1 ± 1 ml homogen angemischt, und in die Halbform eingefüllt. Die Halbform wird zusammengesetzt und der Probekörper nach 10 Minuten bei 23°C entformt. Die Probekörper werden weiter bei 23°C und 50 % relativer Luftfeuchte 24 ± 4 Stunden gelagert und dann vermessen. Die angegebenen Werte wurden durch 5 Parallelmessungen ermittelt.

### Formulierungen:

Die im Rahmen der nachfolgenden Beispiele eingesetzte Basiskomponente wies die folgende Zusammensetzung auf, wobei der Anteil an Weichmacher durch die in den nachfolgenden Tabellen jeweils angegebene Menge an monofunktionellem Monomeren ersetzt wurde:
- 55,5 % difunktionelles Monomeres (Copolymeres aus Ethylenoxid und THF mit einem Molekulargewicht von 6000)
- 14,2 % Fett (Trisacylester des Glycerins nichttierischen Ursprungs)
- 18,1 % Weichmacher (Dibenzyltoluol)
- 12,2 % Füllstoff (Diatomeenerde).

1. Test mit variierenden Anteilen an monofunktionalen Monomeren
Katalysatorkomponente: Zink-Salz der p-Toluolsulfonsäure
Basiskomponente B: Substitution von Weichmacher durch monofunktionales Monomer
Verwendetes monofunktionales Monomer: tertiäres Butanol funktionalisiert mit Ethylenimin

**Tabelle 1:**

| **Verwendete Monomere** | | **Ethylenimingruppen in 100 g Basiskomponente** | **Shore-Härte A nach** | | | |
|---|---|---|---|---|---|---|
| **Monofunktionales Monomeres** | **Difunktionales Monomeres** | | **10 min** | **15 min** | **30 min** | **24 h** |
| 1,55 % | 55,0 % | 2,50 10⁻² mol | 37 | 43 | 49 | 54 |
| 1,16 % | 55,0 % | 2,32 10⁻² mol | 36 | 43 | 49 | 54 |
| 0,78 % | 55,0 % | 2,14 10⁻² mol | 35 | 42 | 48 | 54 |
| 0,39 % | 55,0 % | 1,96 10⁻² mol | 33 | 41 | 48 | 54 |
| 0 % | 55,0 % | 1,77 10⁻² mol | 31 | 40 | 48 | 54 |

Verwendetes monofunktionales Monomeres: Polypropylenglykolmonobutylether funktionalisiert mit Ethylenimin, M =1000 (vor der Funktionalisierung):

**Tabelle 2**

| **Verwendete Monomere** | | **Ethylenimin-Gruppen in 100 g Basiskomponente** | **Shore-Härte A nach** | | | |
|---|---|---|---|---|---|---|
| **Monofunktionales Monomeres** | **Difunktionales Monomeres** | | **10 min** | **15 min** | **30 min** | **24 h** |
| 10,0 % | 55,0 % | 2,50 10⁻² mol | 35 | 42 | 48 | 55 |
| 7,5 % | 55,0 % | 2,32 10⁻² mol | 34 | 42 | 48 | 55 |
| 5,0 % | 55,0 % | 2,14 10⁻² mol | 34 | 42 | 49 | 55 |
| 2,5 % | 55,0 % | 1,96 10⁻² mol | 32 | 41 | 48 | 54 |
| 0 % | 55,0 % | 1,77 10⁻² mol | 31 | 41 | 48 | 54 |

**Tabelle 3**

| **Verwendete Monomere** | | **Viskosität** | | | |
|---|---|---|---|---|---|
| **Monofunktionales Monomeres** | **Difunktionales Monomeres** | **γ̇ = 20.1/s** | **γ̇ = 50 1/s** | **Zugfestigkeit** | **Dehnbarkeit vor Bruch** |
| 0,0 % | 55,0 % | 165 Pas | 105 Pas | 1,21 ± 0,08 MPa | 95 % |
| 10,0 % | 55,0 % | 120 Pas | 85 Pas | 1,32 ± 0,08 MPa | 92 % |
| 17,6 % | 55,0 % | 135 Pas | 95 Pas | 1,38 ± 0,03 MPa | 99 % |

2. Test mit variierenden Anteilen an monofunktionalen Monomeren und Einstellen des Anteils an Polymerisationsinitiator (Molares Verhältnis von Ethylenimin : Anion des Polymerisationsinitiators = 1,4 : 1,0)
a) Katalysatorkomponente: Zink-Salz der p-Toluolsulfonsäure
Basiskomponente: Substitution von Weichmacher durch monofunktionales Monomer
Verwendetes monofunktionales Monomer: Polypropylenglykolmonobutylether funktionalisiert mit Ethylenimin, M = 1000 (vor der Funlctionalisierung):

**Tabelle 4**

| **Verwendete Monomere** | | **Shore-Härte A nach** | | | | | |
|---|---|---|---|---|---|---|---|
| **Monofunktionales Monomeres** | **Difunktionales Monomeres** | **6 min** | **8 min** | **10 min** | **15 min** | **30 min** | **24 h** |
| 10,0% | 55,0 % | 29 | 40 | 45 | 52 | 56 | 58 |
| 7,5 % | 55,0 % | 25 | 36 | 42 | 49 | 55 | 57 |
| 5,0 % | 55,0 % | 20 | 34 | 39 | 47 | 53 | 56 |
| 2,5 % | 55,0 % | 16 | 28 | 35 | 44 | 51 | 56 |
| 0 % | 55,0 % | 11 | 19 | 28 | 38 | 47 | 55 |

b) Katalysatorkomponente: Dodecylbenzensulfonsäuresalz Basiskomponente: Substitution von Weichmacher gegen monofunktionales Monomer
Verwendetes monofunktionales Monomer: Polypropylenglykolmonobutylether funktionalisiert mit Ethylenimin, M = 1000 (vor der Funktionalisierung):

**Tabelle 5:**

| **Verwendete Monomere** | | **Shore-Härte A nach** | | | | | |
|---|---|---|---|---|---|---|---|
| **Monofunktionales Monomeres** | **Di-funktionales Monomeres** | **6 min** | **8 min** | **10 min** | **15 min** | **30 min** | **24 h** |
| 10,0 % | 55,0 % | 40 | 48 | 51 | 56 | 57 | 58 |
| 7,5 % | 55,0 % | 40 | 47 | 51 | 54 | 55 | 57 |
| 5,0 % | 55,0 % | 39 | 45 | 49 | 53 | 54 | 57 |
| 2,5 % | 55,0 % | 37 | 44 | 48 | 53 | 54 | 57 |
| 0 % | 55,0 % | 31 | 42 | 46 | 51 | 54 | 56 |

c) Katalysatorkomponente: p-Toluolsulfonsäure
Basiskomponente: Substitution von Weichmacher durch monofunktionales Monomeres
Verwendetes monofunktionales Monomer: Polypropylenglykolmonobutylether funktionalisiert mit Ethylenimin, M = 1000 (vor der Funktionalisierung):

**Tabelle 6:**

| **Verwendete Monomere** | | **Shore-Härte A nach** | | | | | |
|---|---|---|---|---|---|---|---|
| **Monofunktionales Monomeres** | **Difunktionales Monomeres** | **6 min** | **8 min** | **10 min** | **15 min** | **30 min** | **24 h** |
| 10,0 % | 55,0 % | 18 | 25 | 30 | 39 | 44 | 50 |
| 7,5 % | 55,0 % | 19 | 26 | 32 | 38 | 43 | 49 |
| 5,0 % | 55,0 % | 17 | 25 | 30 | 38 | 43 | 49 |
| 2,5 % | 55,0 % | 19 | 26 | 32 | 39 | 45 | 50 |
| 0 % | 55,0 % | 12 | 20 | 26 | 33 | 41 | 48 |

d) Katalysatorkomponente: Sulfoniumsalz Tetrafluoroborat
Basiskomponente: Substitution von Weichmacher durch monofunktionales Monomer
Verwendetes monofunktionales Monomer: Polypropylenglykolmonobutylether funktionalisiert mit Ethylenimin, M = 1000 (vor der Funktionalisierung):

| **Verwendete Monomere** | | **Shore-Härte A nach** | | | | | |
|---|---|---|---|---|---|---|---|
| **Monofunktionales Monomeres** | **Difunktionales Monomeres** | **6 min** | **8 min** | **10 min** | **15 min** | **30 min** | **24 h** |
| 10,0 % | 55,0 % | 37 | 44 | 46 | 49 | 50 | 50 |
| 7,5 % | 55,0 % | 35 | 41 | 45 | 48 | 49 | 49 |
| 5,0 % | 55,0 % | 34 | 40 | 44 | 47 | 48 | 49 |
| 2,5 % | 55,0 % | 32 | 38 | 43 | 46 | 48 | 49 |
| 0 % | 55,0 % | 32 | 39 | 41 | 45 | 47 | 47 |

## Patentansprüche

1. Zusammensetzung, mindestens enthaltend zwei Komponenten Z1 und Z2, wobei die Zusammensetzung
a) als Komponente Z1 mindestens ein Polyadditionsprodukt oder ein Polykondensationsprodukt mit durchschnittlich 2 Aziridinogruppen oder mehr und einem Molekulargewicht von mindestens 1000 und
b) als Komponente Z2 mindestens eine Verbindung mit 1 Aziridinogruppe enthält, wobei mindestens eine Verbindung gemäß Komponente Z2 sich in ihrem chemischen Aufbau von mindestens einer Verbindung gemäß Komponente Z1 in mindestens einem weiteren Merkmal als der Zahl der Aziridinogruppen unterscheidet, wobei der Unterschied mindestens in einem oder zwei oder mehr der nachfolgenden weiteren Merkmale von Komponente Z1 besteht:
i) Zahlenmittel des Molekulargewichts,
ii) Gewichtsmittel des Molekulargewichts,
iii) Polydispersität,
iv) Zusammensetzung des Polymerrückgrats,
v) Endgruppen.

2. Zusammensetzung nach Anspruch 1, **dadurch gekennzeichnet, dass** sie als Komponente Z1 mindestens ein Polymeres ausgewählt aus der Gruppe, bestehend aus Polyethern, Polyestern, Polyurethanen oder Polydimethylsiloxanen enthält.

3. Zusammensetzung nach Anspruch 1 oder 2, **dadurch gekennzeichnet, dass** sie als Komponente Z1 einen Polyether mit mindestens einem Anteil an Tetrahydrofuraneinheiten enthält.

4. Zusammensetzung nach einem der Ansprüche 1 bis 3, **dadurch gekennzeichnet, dass** sie als Komponente Z2 eine Verbindung ausgewählt aus der Gruppe bestehend aus Polyethern, Polyestern, Polyurethanen oder Polydimethylsiloxanen enthält.

5. Zusammensetzung nach einem der Ansprüche 1 bis 4, **dadurch gekennzeichnet, dass** sie als Komponente Z2 eine Verbindung mit einem Molekulargewicht von 300 oder mehr enthält.

6. Zusammensetzung nach einem der Ansprüche 1 bis 5, **dadurch gekennzeichnet, dass** sie einen Zusatzstoff oder ein Gemisch aus zwei oder mehr Zusatzstoffen enthält.

7. Verfahren zur Herstellung einer Zusammensetzung gemäß einem der Ansprüche 1 bis 6, bei dem zwei Komponenten Z1 und Z2 vermischt werden, wobei
a) als Komponente Z1 mindestens ein Polyadditionsprodukt oder ein Polykondensationsprodukt mit durchschnittlich 2 Aziridinogruppen oder mehr und einem Molekulargewicht von mindestens 1000 und
b) als Komponente Z2 mindestens eine Verbindung mit 1 Aziridinogruppe eingesetzt wird, und wobei mindestens eine Verbindung gemäß Komponente Z2 sich in ihrem chemischen Aufbau von mindestens einer Verbindung gemäß Komponente Z1 in mindestens einem weiteren Merkmal als der Zahl der Aziridinogruppen unterscheidet, wobei der Unterschied mindestens in einem oder zwei oder mehr der nachfolgenden weiteren Merkmale von Komponente Z1 besteht:
i) Zahlenmittel des Molekulargewichts,
ii) Gewichtsmittel des Molekulargewichts,
iii) Polydispersität,
iv) Zusammensetzung des Polymerrückgrats,
v) Endgruppen.

8. Dentalmasse, umfassend mindestens eine Basiskomponente B und eine Katalysatorkomponente K, wobei die Basiskomponente B mindestens eine Zusammensetzung gemäß einem der Ansprüche 1 bis 6 und die Katalysatorkomponente K mindestens einen Katalysator zur Vernetzung zumindest eines Teils der Basiskomponente B enthält.

9. Dentalmasse nach Anspruch 8, **dadurch gekennzeichnet, dass** sie nach Mischen der Basiskomponente B und der Katalysatorkomponente K bei Raumtemperatur innerhalb eines Zeitraums von 20 Minuten oder weniger eine Shore-Härte A von mindestens 80% des nach 24 Stunden erreichten Werts für die Shore-Härte A aufweist.

10. Verwendung einer Zusammensetzung gemäß einem der Ansprüche 1 bis 6 als Basiskomponente B für Beschichtungen, Abformmassen, Dichtungen oder Dentalabdruckmassen.

11. Verwendung einer Verbindung mit 1 Aziridinogruppe zur Beschleunigung der Abbindegeschwindigkeit von Dentalmassen gemäß einem der Ansprüche 8 oder 9.

12. Kit zur Herstellung von Dentalmassen, umfassend mindestens eine Zusammensetzung gemäß einem der Ansprüche 1 bis 6 als Basiskomponente B und mindestens eine einen Katalysator zur Vernetzung zumindest eines Teils der Basiskomponente B enthaltende Katalysatorkomponente K, wobei die Komponenten B und K voneinander getrennt vorliegen.

13. Behältnisse und Mischvorrichtungen, enthaltend eine Dentalmasse gemäß Anspruch 8 oder 9.

## Claims

1. Composition comprising at least the two components Z1 and Z2, the composition comprising,
a) as component Z1, at least one polyaddition product or polycondensation product having on average 2 aziridino groups or more and a molecular weight of at least 1000 and,
b) as component Z2, at least one compound having 1 aziridino group, at least one compound according to component Z2 differing, in its chemical make-up, from at least one compound according to component Z1 in at least one further feature other than the number of the aziridino groups, the difference consisting at least in one or two or more of the following further features of component Z1:
i) number average of the molecular weight,
ii) weight average of the molecular weight,
iii) polydispersity,
iv) composition of the polymer backbone,
v) end groups.

2. Composition according to Claim 1, **characterised in that** it comprises, as component Z1, at least one polymer selected from the group consisting of polyethers, polyesters, polyurethanes and polydimethylsiloxanes.

3. Composition according to Claim 1 or 2, **characterised in that** it comprises, as component Z1, a polyether having at least a proportion of tetrahydrofuran units.

4. Composition according to one of Claims 1 to 3, **characterised in that** it comprises, as component Z2, a compound selected from the group consisting of polyethers, polyesters, polyurethanes and polydimethylsiloxanes.

5. Composition according to one of Claims 1 to 4, **characterised in that** it comprises, as component Z2, a compound having a moleculr weight of 300 or more.

6. Composition according to one of Claims 1 to 5, **characterised in that** it comprises an additive or a mixture of two or more additives.

7. Process for the preparation of a composition according to one of Claims 1 to 6, wherein two components Z1 and Z2 are mixed together, there being used,
a) as component Z1, at least one polyaddition product or polycondensation product having on average 2 aziridino groups or more and a molecular weight of at least 1000 and,
b) as component Z2, at least one compound having 1 aziridino group, and at least one compound according to component Z2 differing, in its chemical make-up, from at least one compound according to component Z1 in at least one further feature other than the number of the aziridino groups, the difference consisting at least in one or two or more of the following further features of component Z1:
i) number average of the molecular weight,
ii) weight average of the molecular weight,
iii) polydispersity,
iv) composition of the polymer backbone,
v) end groups.

8. Dental material comprising at least one basic component B and one catalyst component K, basic component B comprising at least one composition according to one of Claims 1 to 6 and catalyst component K comprising at least one catalyst for the cross-linking of at least part of basic component B.

9. Dental material according to Claim 9, **characterised in that** it has, after mixing of basic component B and catalyst component K at room temperature, within a period of 20 minutes or less, a Shore A hardness of at least 80% of the value of Shore A hardness reached after 24 hours.

10. Use of a composition according to one of Claims 1 to 6 as basic component B for coatings, impression materials, seals or dental moulding materials.

11. Use of a compound having 1 aziridino group in accelerating the setting rate of dental materials according to one of Claims 8 and 9.

12. Kit for producing dental materials, comprising at least one composition according to one of Claims 1 to 6 as basic component B and at least one catalyst component K comprising a catalyst for the cross-linking of at least part of basic component B, the components B and K being present separated from one another.

13. Containers and mixing devices containing a dental material according to Claim 8 or 9.

## Revendications

1. Composition contenant au moins deux composants Z1 et Z2, où la composition contient
a) en tant que composant Z1, au moins un produit de polyaddition ou un produit de polycondensation comportant en moyenne 2 groupes aziridino ou plus et ayant un poids moléculaire d'au moins 1000, et
b) en tant que composant Z2, au moins un composé comportant 1 groupe aziridino, où au moins un composé selon le composant Z2 se distingue, dans sa structure chimique, d'au moins un composé selon le composant Z1 en au moins une autre caractéristique que le nombre des groupes aziridino, la différence consistant en au moins une ou deux ou plus de deux des autres caractéristiques suivantes du composant Z1 :
i) moyenne numérique du poids moléculaire,
ii) moyenne pondérale du poids moléculaire,
iii) polydispersivité,
iv) composition du squelette polymère,
v) groupes terminaux.

2. Composition suivant la revendication 1, **caractérisée en ce qu'**elle contient en tant que composant Z1 au moins un polymère choisi dans le groupe formé par des polyéthers, des polyesters, des polyuréthannes ou des polydiméthylsiloxanes.

3. Composition suivant la revendication 1 ou 2, **caractérisée en ce qu'**elle contient en tant que composant Z1 un polyéther comportant au moins une fraction d'unités de tétrahydrofuranne.

4. Composition suivant l'une quelconque des revendications 1 à 3, **caractérisée en ce qu'**elle contient en tant que composant Z2 un composé choisi dans le groupe formé par des polyéthers, des polyesters, des polyuréthannes ou des polydiméthylsiloxanes.

5. Composition suivant l'une quelconque des revendications 1 à 4, **caractérisée en ce qu'**elle contient en tant que composant Z2 un composé d'un poids moléculaire de 300 ou plus.

6. Composition suivant l'une quelconque des revendications 1 à 5; **caractérisée en ce qu'**elle contient un additif ou un mélange de deux ou plus de deux additifs.

7. Procédé de préparation d'une composition suivant l'une quelconque des revendications 1 à 6, dans lequel on mélange deux composants Z1 et Z2, et où l'on met en oeuvre :
a) en tant que composant Z1, au moins un produit de polyaddition ou un produit de polycondensation comportant en moyenne 2 groupes aziridino ou plus et ayant un poids moléculaire d'au moins 1000, et
b) en tant que composant Z2, au moins un composé comportant 1 groupe aziridino, où au moins un composé selon le composant Z2 se distingue, dans sa structure chimique, d'au moins un composé selon le composant Z1 en au moins une autre caractéristique que le nombre des groupes aziridino, la différence consistant en au moins une ou deux ou plus de deux des autres caractéristiques suivantes du composant Z1 :
i) moyenne numérique du poids moléculaire,
ii) moyenne pondérale du poids moléculaire,
iii) polydispersivité,
iv) composition du squelette polymère,
v) groupes terminaux.

8. Masse dentaire comprenant au moins un composant de base B et un composant catalyseur K, où le composant de base B contient au moins une composition suivant l'une quelconque des revendications 1 à 6 et le composant catalyseur K au moins un catalyseur pour la réticulation d'au moins une partie du composant de base B.

9. Masse dentaire suivant la revendication 8, **caractérisée en ce que** qu'elle présente, après mélange du composant de base B et du composant catalyseur K, à la température ambiante et en un laps de temps de 20 minutes ou moins, une dureté Shore A d'au moins 80% de la valeur atteinte après 24 heures pour la dureté Shore A.

10. Utilisation d'une composition suivant l'une quelconque des revendications 1 à 6 en tant que composant de base B pour des revêtements, des masses de moulage, des étanchements ou des masses d'empreintes dentaires.

11. Utilisation d'un composé comportant 1 groupe aziridino pour accélérer la vitesse de prise de masses dentaires suivant l'une quelconque des revendications 8 ou 9.

12. Trousse de préparation de masses dentaires, comprenant au moins une composition suivant l'une quelconque des revendications 1 à 6 en tant que composant de base B et au moins un composant catalyseur K contenant un catalyseur pour la réticulation d'au moins une partie du composant de base B, les composants B et K étant séparés l'un de l'autre.

13. Récipients et dispositifs de mélange contenant une masse dentaire suivant la revendication 8 ou 9.
